(19) Europäisches Patentamt
European Patent Office
Office européen des brevets

(11) Veröffentlichungsnummer: **0 355 578 A1**

# (12) EUROPÄISCHE PATENTANMELDUNG

(21) Anmeldenummer: **89114689.6**

(22) Anmeldetag: **09.08.89**

(51) Int. Cl.[4]: **C07C 333/04**

(30) Priorität: **24.08.88 DE 3828643**

(43) Veröffentlichungstag der Anmeldung:
**28.02.90 Patentblatt 90/09**

(84) Benannte Vertragsstaaten:
**CH DE FR GB IT LI**

(71) Anmelder: **CASSELLA Aktiengesellschaft**
**Hanauer Landstrasse 526**
**D-6000 Frankfurt am Main 61(DE)**

(72) Erfinder: **Becherer, Johannes, Dr.**
**Weidenseestrasse 8**
**D-6457 Maintal 2(DE)**

(74) Vertreter: **Urbach, Hans-Georg, Dr. et al**
**Hanauer Landstrasse 526**
**D-6000 Frankfurt am Main 61(DE)**

## (54) Verfahren zur Herstellung von N,N-Dimethylthiocarbamoylchlorid.

(57) Die Erfindung betrifft ein Verfahren zur Herstellung von N,N-Dimethylthiocarbamoylchlorid durch Umsetzung von Tetramethylthiuramdisulfid mit einem Chlorierungsmittel, wobei als Reaktionsmedium geschmolzenes N,N-Dimethylthiocarbamoylchlorid verwendet wird.

EP 0 355 578 A1

Xerox Copy Centre

## Verfahren zur Herstellung von N,N-Dimethylthiocarbamoylchlorid

Die vorliegende Erfindung betrifft ein Verfahren zur Herstellung von N,N-Dimethylthiocarbamoylchlorid durch Umsetzung von Tetramethylthiuramdisulfid mit einem Chlorierungsmittel.

Es sind bereits verschiedene Wege zur Herstellung von N,N-Dimethylthiocarbamoylchlorid beschrieben worden (Houben-Weyl, Methoden der Organischen Chemie, 4. Auflage, 1983, Band E 4, Seiten 415 ff), die jedoch alle mit teils erheblichen Nachteilen behaftet sind, sei es, daß die Ausbeuten schlecht sind, schwer zu entsorgende Nebenprodukte entstehen oder daß sie von teuren bzw. in technischem Maßstab nicht verfügbaren Ausgangsverbindungen ausgehen.

Auch die bekannten Verfahren, die von Tetramethylthiuramdisulfid und einem Chlorierungsmittel ausgehen, bringen im technischen Maßstab schwerwiegende Nachteile aus ökonomischer und ökologischer Sicht mit sich. So wird hier als Reaktionsmedium Tetrachlorkohlenstoff eingesetzt (z.B. Houben-Weyl, Band E 4, Seite 419; Yen, Cava J.Org.Chem. 48, 1449 (1983)). Dies führt zu schlechten Raumausbeuten und dadurch bedingt zu hohen Investitionskosten, außerdem zu einem vermehrten Energiebedarf für das Abdestillieren des Lösungsmittels und zu kostspieligen Maßnahmen, um Lösungsmittelverluste aus Umweltschutzgründen zu minimieren. In Org. Synth.Coll., Vol.IV, Seite 307, ist ein Verfahren zur Herstellung von N,N-Diethylthiocarbamoylchlorid beschrieben, das lösungsmittelfreies Arbeiten erlaubt, indem eine äquivalente Menge Chlor in geschmolzenes Tetraethylthiuramdisulfid eingeleitet wird. Dieses Verfahren ist allerdings auf die Synthese von N,N-Dimethylthiocarbamoylchlorid aufgrund des hohen Schmelzpunktes von Tetramethylthiuramdisulfid (ca. 150°C) nicht übertragbar. es wurde nun versucht, ein Verfahren aufzufinden, das die genannten Nachteile nicht aufweist.

Gegenstand vorliegender Erfindung ist demnach ein Verfahren zur Herstellung von N,N-Dimethylthiocarbamoylchlorid der Formel I

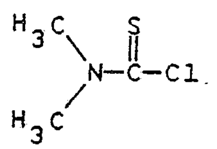

(I)

durch Umsetzung von Tetramethylthiuramdisulfid der Formel II

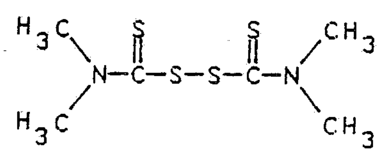

(II)

mit Chlorierungsmitteln in einer Menge, die gerade für die Chlorierung der Verbindung der Formel II ausreicht, dadurch gekennzeichnet, daß als Reaktionsmedium geschmolzenes N,N-Dimethylthiocarbamoylchlorid der Formel I eingesetzt wird.

Da die Verbindung der Formel I unter den Reaktionsbedingungen sehr leicht unter exothermer Reaktion weiterchloriert wird, wobei 1,1,1-Trichlortrimethylamin der Formel III

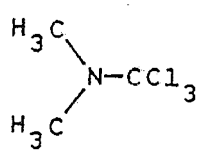

(III)

entsteht (Houben-Weyl, Methoden der Org. Chemie, 4. Auflage 1983, Bd. E 4, Seite 655; Yarovenko, Vasil'eva, Zh. Obshch. Kim 29 3786 (1959) CA 54 19466; Senning, Chem. Rev. 65 385 (1965)), ist es besonders überraschend, daß im erfindungsgemäßen Verfahren selektiv die Verbindung der Formel II chloriert wird und die Verbindung der Formel I trotz des immer vorliegenden großen Überschusses nicht

angegriffen wird, so daß dieselben guten Ausbeuten erhalten werden wie beim Arbeiten in Verdünnung mit einem Lösungsmittel.

Als Chlorierungsmittel werden bevorzugt elementares Chlor, Schwefeldichlorid $SCl_2$, Dischwefeldichlorid $S_2Cl_2$ oder Sulfurylchlorid $SO_2Cl_2$ eingesetzt. Selbstverständlich können auch Gemische verschiedener Chlorierungsmittel eingesetzt werden, sofern diese nicht miteinander reagieren.

Die Chlorierungsmittel werden in Mengen eingesetzt, die gerade für die Chlorierung der Verbindung der Formel II ausreichen. Je nach Reinheitsgrad der eingesetzten Verbindung der Formel II muß zur Erzielung optimaler Ausbeuten der Chlorierungsmittelverbrauch der darin enthaltenen Verunreinigungen berücksichtigt werden. Abweichungen im Bereich einiger Prozent sind möglich. Im Falle der genannten Reagenzien Chlor, Schwefeldichlorid, Dischwefeldichlorid und Sulfurylchlorid heißt das, daß sie in Mengen von 0,95 bis 1,2 Mol pro Mol Verbindung der Formel II eingesetzt werden.

Das als Reaktionsmedium verwendete geschmolzene N,N-Dimethylthiocarbamoylchlorid der Formel I wird bei einer laufenden Produktion bevorzugt in Form eines rohen Reaktionsgemisches eines vorhergehenden Ansatzes eingesetzt. Die Ausgangsverbindung Tetramethylthiuramdisulfid der Formel II ist eine käufliche Verbindung.

Das erfindungsgemäße Verfahren wird vorteilhafterweise bei Temperaturen zwischen 35°C und 120°C, bevorzugt zwischen 40°C und 60°C, ausgeführt.

In einer bevorzugten Ausführungsform des erfindungsgemäßen Verfahrens wird eine geringe Menge der Verbindung der Formel I oberhalb ihres Erstarrungspunktes, bevorzugt bei Temperaturen von 35 bis 120°C, besonders bevorzugt 40 bis 60°C, vorgelegt, unter Rühren so viel Verbindung der Formel II eingetragen, daß die Suspension noch rührfähig bleibt, und unter weiterem Rühren die entsprechende Menge des Chlorierungsmittels zudosiert, wobei die Ausgangsverbindung der Formel II in das Produkt der Formel I überführt wird und das Gemisch wieder dünnflüssig wird. Dann können erneut Ausgangsverbindung und anschließend Chlorierungsmittel zudosiert werden. Dies wird so lange wiederholt, bis die gewünschte Ansatzgröße erreicht bzw. das Reaktionsgefäß voll ist.

Die Mindestmenge an geschmolzener Verbindung der Formel I, die zu Beginn der Reaktion vorgelegt werden muß, wird im wesentlichen durch die Form des Reaktionsgefäßes bestimmt. Bezogen auf die Gesamtmenge der zum Einsatz kommenden Ausgangsverbindung der Formel II, können vorteilhafterweise 5 bis 50 Gew.%, besonders bevorzugt 10 bis 25 Gew.%, vorgelegt werden. Eine größere Menge ist normalerweise nicht erforderlich.

Die Verbindung der Formel II kann in die vorgelegte Schmelze portionsweise oder kontinuierlich über einen Zeitraum von vorzugsweise 0,5 bis 10 Stunden eingetragen werden. Die Zugabe des Chlorierungsmittels ist darauf bezogen so abgestimmt, daß das Reaktionsgemisch immer gut rührbar bleibt, aber nie Chlorierungsmittel im Überschuß vorliegt.

Während der ganzen Reaktion wird die Temperatur oberhalb des Erstarrungspunktes des Reaktionsgemisches, bevorzugt bei 40 bis 60°C, gehalten. Höhere Temperaturen, etwa bis 120°C, sind auch möglich, führen aber zu Ausbeuteverlusten.

Die Aufarbeitung des Reaktionsgemisches erfolgt vorzugsweise durch Destillation unter vermindertem Druck. Es ist auch möglich, vom abgeschiedenen Schwefel abzudekantieren oder abzufiltrieren und das Produkt umzukristallisieren.

Das erfindungsgemäße Verfahren liefert die Verbindung der Formel I in ausgezeichneter Ausbeute und hoher Reinheit. Die Raumausbeute der Reaktion ist sehr hoch. Da kein Lösungsmittel abdestilliert werden muß, ist der Energiebedarf gering. Eine Umweltbelastung durch Lösungsmittel entfällt.

N,N-Dimethylthiocarbamoylchlorid der Formel I ist ein wichtiges Reagenz zur Einführung von Thiolgruppen in aromatische Verbindungen durch die Newman-Kwart-Umlagerung (siehe z.B. US-P 3,476,791 oder EP-A 269.434).

Die folgenden Beispiele erläutern das erfindungsgemäße Verfahren:

Beispiel 1

In einem 500-ml-Vierhalskolben mit Rührer, Innenthermometer, Gaseinleitungsrohr und Blasenzähler zum Druckausgleich werden 70 g N,N-Dimethylthiocarbamoylchlorid bei 50°C aufgeschmolzen. Dann werden abwechselnd 97%iges Tetramethylthiuramdisulfid und Chlor unter Rühren nach folgendem Schema eingetragen bzw. in den Gasraum geleitet, wobei die Temperatur durch leichte Außenkühlung zwischen 45° und 55°C gehalten wird:

a) 40 g N,N-Dimethylthiocarbamoylchlorid, dann 10 g $Cl_2$ in ca 15 Minuten

b) 60 g N,N-Dimethylthiocarbamoylchlorid, dann 15 g $Cl_2$ in ca. 30 Minuten

c) 90 g N,N-Dimethylthiocarbamoylchlorid, dann 25 g $Cl_2$ in ca. 45 Minuten

d) 90 g N,N-Dimethylthiocarbamoylchlorid, dann 30 g $Cl_2$ in ca. 45 Minuten

e) 90 g N,N-Dimethylthiocarbamoylchlorid, dann 30 g $Cl_2$ in ca. 45 Minuten Gesamteinsatzmenge: 370 g Tetramethylthiuramdisulfid (97%ig; entspricht 1,5 Mol) und 110 g $Cl_2$ (1,55 Mol). Das Reaktionsgemisch, welches aus einer dünnflüssigen Produktphase und einer zähflüssigen Schwefelphase besteht, wird bei ca. 20 mbar destilliert, wobei bei etwa 110°C ca 424 g N,N-Dimethylthiocarbamoylchlorid über gehen.

Ausbeute, abzüglich der eingesetzten 70 g: 354 g

Gaschromatographische Reinheit: 96,3% (Flächenprozent, FID)

Erstarrungspunkt: 41,0°C

Ausbeute: 92% der Theorie

Beispiel 2

Verfährt man wie in Beispiel 1, leitet jedoch kein Chlor ein, sondern tropft in insgesamt einer Stunde 216 g (1,6 Mol) $S_2Cl_2$ in Portionen zu 20, 30, 45, 55 und 66 g zu, rührt eine halbe Stunde nach und destilliert anschliessend, so erhält man abzüglich der eingesetzten 70 g ca. 377 g Produkt.

Gaschromatographische Reinheit: 97,7%

Erstarrungspunkt: 40,4°C

Ausbeute: 99% der Theorie

Beispiel 3

Verfährt man wie in Beispiel 2 mit dem Unterschied, daß statt $S_2Cl_2$ 160 g (1,55 Mol) $SCl_2$ in Portionen von 15, 20, 40 und 45 g zugetropft werden, so erhält man abzüglich der eingesetzten 70 g ca. 365 g Produkt.

Gaschromatographische Reinheit: 97,3%

Erstarrungspunkt: 40,6°C

Ausbeute: 96% der Theorie

Beispiel 4

Verfährt man wie in Beispiel 2 mit dem Unterschied, daß statt $S_2Cl_2$ 210 g (1,55 Mol) $SO_2Cl_2$ in Portionen von 20, 30, 45, 55 und 60 g zugetropft werden, so erhält man abzüglich der eingesetzten 70 g ca. 363 g Produkt.

Gaschromatographische Reinheit: 97,0%

Erstarrungspunkt: 40,6°C

Ausbeute: 95% der Theorie

Beispiel 5

Verfährt man wie in Beispiel 1, legt jedoch nicht reines N,N-Dimethylthiocarbamoylchlorid, sondern rohes, undestilliertes Reaktionsgemisch eines vorhergehenden Ansatzes, welches 70 g des Produktes enthält, vor, so erhält man das Produkt in gleicher Ausbeute und Reinheit.

Beispiel 6

Verfährt man wie in Beispiel 2, trägt jedoch das Tetramethylthiuramdisulfid nicht in Portionen, sondern kontinuierlich über eine Feststoffdosierschnecke im Verlaufe einer Stunde ein und tropft das $S_2Cl_2$ gleichmäßig mit einer Zeitverzögerung von ca. 7 Minuten ebenfalls im Verlaufe von einer Stunde zu, so erhält man das Produkt in gleicher Ausbeute und Reinheit.

**Ansprüche**

1. Verfahren zur Herstellung von N,N-Dimethylthiocarbamoylchlorid der Formel I

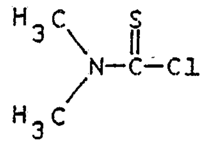

(I)

durch Umsetzung von Tetramethylthiuramdisulfid der Formel II

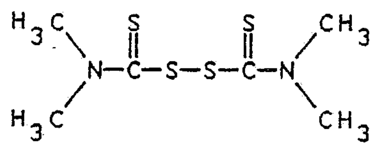

(II)

mit Chlorierungsmitteln in einer Menge, die gerade für die Chlorierung der Verbindung der Formel II ausreicht, dadurch gekennzeichnet, daß als Reaktionsmedium geschmolzenes N,N-Dimethylthiocarbamoylchlorid der Formel I eingesetzt wird.

2. Verfahren gemäß Anspruch 1, dadurch gekennzeichnet, daß als Chlorierungsmittel Chlor, Schwefeldichlorid, Dischwefeldichlorid oder Sulfurylchlorid eingesetzt werden.

3. Verfahren gemäß Anspruch 1 und/oder 2, dadurch gekennzeichnet, daß das als Reaktionsmedium verwendete geschmolzene N,N-Dimethylthiocarbamoylchlorid der Formel I in Form eines rohen Reaktionsgemisches eines vorhergehenden Ansatzes eingesetzt wird.

4. Verfahren gemäß einem oder mehreren der Ansprüche 1 bis 3, dadurch gekennzeichnet, daß eine geringe Menge der Verbindung der Formel I oberhalb ihres Erstarrungspunktes vorgelegt wird, dann unter Rühren soviel Verbindung der Formel II eingetragen wird, daß die Suspension noch rührfähig bleibt, dann die entsprechende Menge Chlorierungsmittel zudosiert wird und dieser Vorgang so lange wiederholt wird, bis die gewünschte Ansatzgröße erreicht ist.

5. Verfahren gemäß Anspruch 4, dadurch gekennzeichnet, daß die Verbindung der Formel II kontinuierlich eingetragen und die Zugabe des Chlorierungsmittels darauf abgestimmt wird.

6. Verfahren gemäß Anspruch 4 und/oder 5, dadurch gekennzeichnet, daß die Umsetzung bei Temperaturen von 40 bis 60°C ausgeführt wird.

7. Verfahren gemäß einem oder mehreren der Ansprüche 4 bis 6, dadurch gekennzeichnet, daß die Verbindung der Formel I in Mengen von 5 bis 50 Gew.%, bezogen auf die Gesamtmenge der zum Einsatz kommenden Verbindung der Formel II, vorgelegt wird.

Europäisches Patentamt

# EUROPÄISCHER RECHERCHENBERICHT

Nummer der Anmeldung

EP 89 11 4689

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | "Organic syntheses", Band 35, 1955, Seiten 55-58, John Wiley & Sons, Inc., New York, US; R.H. GOSHORN et al.: "Diethylthiocarbamyl chloride" * Seiten 56-57, "note 5" * | 1-7 | C 07 C 333/04 |
| Y | FR-A-1 085 980 (E. JAUL et al.) * Beispiel 3 * | 1-7 | |
| Y | FR-A- 960 006 (SHARPLES CHEMICALS) * Beispiel 10 * | 1-7 | |
| D,A | "Methoden der organischen Chemie", Band E4, 1983, Seiten 415-420, Houben-Weyl, Stuttgart, DE; "Thiocarbamidsäure-halogenide" | 1 | |
| | | | **RECHERCHIERTE SACHGEBIETE (Int. Cl.5)** |
| | | | C 07 C 333/00 |

Der vorliegende Recherchenbericht wurde für alle Patentansprüche erstellt

| Recherchenort | Abschlußdatum der Recherche | Prüfer |
|---|---|---|
| DEN HAAG | 03-11-1989 | WELLS A.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)